# EUROPEAN PATENT SPECIFICATION

(11) **EP 0 744 187 B1**
(45) Date of publication and mention of the grant of the patent: **18.07.2001**
(21) Application number: 96201334.8
(22) Date of filing: 15.05.1996
(51) Int. Cl.: A61M 29/02

(54) **Balloon catheter with balloon protection sheath**
Ballonkatheter mit Ballonschutzumhüllung
Cathéter à ballonnet et gaine protectrice du ballonet

(30) Priority: 22.05.1995 NL 1000413
(43) Date of publication of application: 27.11.1996
(73) Proprietor: CORDIS EUROPA N.V., NL-9301 LJ Roden (NL)
(72) Inventor: Klunder, Rento Willem, 9742 EV Groningen (NL); Van Werven-Franssen, Gerda Hendrika Maria, 9302 EK Roden (NL)
(74) Representative: 't Jong, Bastiaan Jacobus

(56) References cited:
- EP-A- 0 331 040
- EP-A- 0 461 474
- WO-A-84/00113
- WO-A-95/03082
- WO-A-95/11718
- AT-A- 366 904

## Description

The invention relates to a balloon catheter which comprises a tubular basic body with a distal and a proximal end and wherein a balloon member is arranged on the distal end.

Such balloon catheters are generally known (see. e.g. EP-A- 461 474). They serve for example to dilate a stenosis in the blood circulation of a patient. Such a balloon must on the one hand be sufficiently strong to be able to safely withstand the loads occurring during insertion and treatment. On the other hand it is desired that at the position of the balloon, which normally determines the largest diameter of the catheter, the catheter is embodied with the smallest possible diameter so as to enable insertion into the patient through the smallest possible introducer.

With the catheter as characterized in claim 1 these two conflicting requirements are optimally satisfied. In the situation where it is pulled over the balloon using the at least one pull thread, the balloon protection sheath provides a great resistance to damage while still not enlarging the effective diameter of the catheter at the position of the balloon when the catheter is inserted, because in this situation the balloon protection sheath is pushed off the balloon on or into the adjoining portion of the basic body.

After insertion of the catheter in the situation where the balloon protection sheath is pushed off the balloon and thus has the smallest possible diameter, the balloon protection sheath is slid onto the balloon by pulling on the pull thread, whereafter the balloon can be expanded in the usual manner. In the expanded active situation the balloon is optimally protected against mechanical influences from outside.

The balloon protection sheath can be a sleeve slidable over the basic body of the catheter, but in preference the step of claim 2 is applied. The position of the balloon protection sheath is then clearly determined so that reliable operation can be achieved.

The step of claim 3 is preferably applied. By guiding the pull thread through the lumen, the pull thread and the basic body act respectively as the inner cable and the outer cable of a pull cable. A pulling force exerted on the pull thread does not then influence the location of the catheter, nor is there a danger of the pull thread, for instance in a bend, being pressed with force against the wall of the blood vessel, whereby there would be a risk of trauma.

Applying the step of claim 4 ensures that the balloon protection sheath does not already slide over the balloon during insertion of the catheter. The relative movement of the catheter along the wall of the blood vessel shifts the sheath in the direction toward the proximal end, whereby the sheath is held in place clear of the balloon.

According to a further development the step of claim 5 is applied. Herewith the protection sheath can be pulled over as well as pulled off the balloon.

A suitable embodiment is characterized in claim 6. The balloon protection sheath can herein be woven as a nylon stocking so that it can be stretched elastically and, after removal of a force causing the stretching, returns to its original shape, i.e. clear of the balloon member.

The invention will be further elucidated in the following description with reference to the annexed figures.

Figure 1 shows a partial section of a balloon catheter according to the invention at the position of the balloon in a first situation of the protection sheath.

Figure 2 shows the catheter of figure 1 in a second situation.

Figure 3 shows the catheter of figure 1 and 2 with the balloon in inflated state.

Figure 4 shows a view corresponding with figure 3 of an alternative embodiment.

Figure 5 shows an embodiment variant of the embodiment of figure 1.

Figure 6 shows yet another embodiment of the catheter according to the invention.

Figure 7 shows the embodiment of figure 6 with the balloon in inflated state.

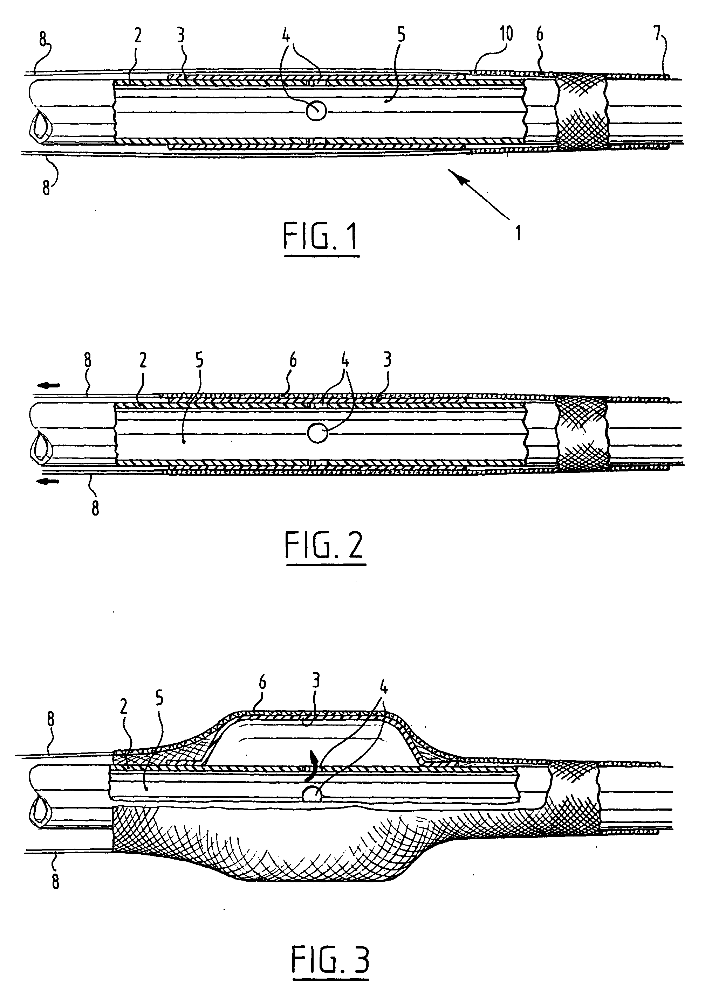

The catheter 1 shown in fig. 1 comprises a tubular basic body 2, a distal end part of which is shown. Arranged round the distal end part is a balloon member 3 which is fixedly and sealingly connected at either end to the outer surface of basic body 2.

Extending in the basic body 2 in this embodiment is one lumen 5 which connects at the proximal end (not shown) to a connecting member through which a fluid under pressure can be supplied. This fluid travels via the apertures 4 in the wall of basic body 2 into the interior of the balloon member 3 which can thereby be expanded.

Close to balloon member 3 an elastic balloon protection sheath 6 is arranged round basic body 2, which sheath can be pulled over the balloon 3 with two pull threads 8 which are fastened to the end of sheath 6 facing toward the balloon member 3 and which extend from this end 10 to the proximal end of the basic body.

The balloon protection sheath 6 is fixedly connected with its opposite end, that is, the end 7 remote from balloon member 3, to the basic body 2. The balloon protection sheath 6 is of elastic material and for instance of material woven in the manner of nylon stockings, so that in released state the sheath 6 is contracted onto the relatively distal end of balloon member 3.

In this situation shown in fig. 1 the catheter 1 has a relatively limited diameter which is effectively no larger than the diameter of the basic body at the position of balloon member 3. The sheath 6 does not lie on balloon member 3, thus causing no enlargement of diameter. Catheter 1 can thus be introduced into a patient by means of an introducer of relatively limited diameter.

As soon as balloon member 3 is arranged at the intended location, for instance at the position of a stenosis, the sheath 6 is pulled over balloon 3 by the treating physician pulling on the end part of the pull threads 8 situated at the proximal end of the catheter. Fluid is subsequently fed under pressure via lumen 5 and apertures 4 therein into the interior of balloon member 3 which thereby expands in the manner shown in fig. 3. The protection sheath 6 situated round the balloon member 3 also expands because it is elastic but at the same time continues to form a protective layer round balloon member 3, so that the actual balloon 3 is protected against damage. In the case of for instance a calcified stenosis, the protection sheath 6 prevents damage to balloon member 3.

Instead of application for dilatation the balloon catheter according to the invention can also be used to arrange a stent or graft. Due to the good protection provided the balloon 3 by protection sheath 6, the chance of damage to the balloon member 3 is also minimal in these applications.

At the end of the treatment the balloon member 3 is again allowed to contract by removing the pressure on the supplied fluid. The sheath 6 likewise contracts due to its elasticity. By releasing the pull threads 8 the sheath 6 can slide back again into its original situation as shown in fig. 1. In this situation the catheter can be taken out.

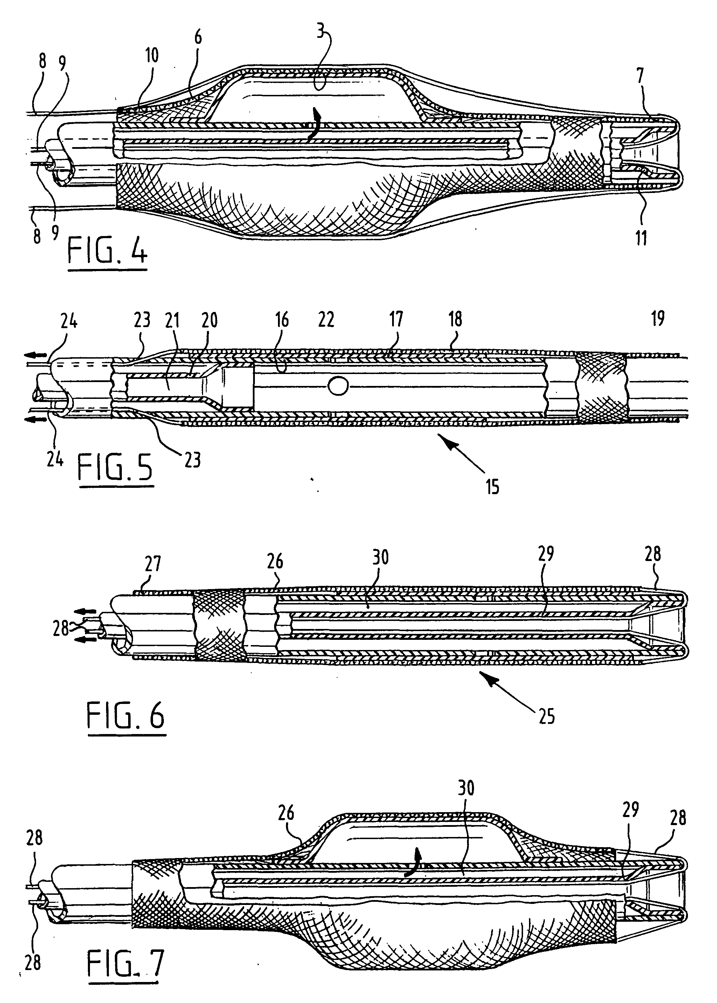

In the embodiment shown in fig. 1-3 the protection sheath 6 is, as stated, of elastically stretchable material. The sheath can however also be manufactured from material which is not elastically stretchable in longitudinal direction. In that case the sheath can be moved back again to its starting position by an additional set of pull threads acting in the opposing direction. Such an embodiment is shown in fig. 4. The pull threads 9 are herein fixed at substantially the same place on sheath 6 as pull threads 8. Pull threads 9 extend first in the direction of the distal end of the catheter shown on the right in fig. 4 and are then guided inside an inner tubular member received in basic body 2. Pull threads 9 run through a lumen of this inner tubular member 11 to the proximal end where they can be controlled by the treating physician. Although here the right-hand end of the protection sheath 6 is fixedly connected to the basic body, it is of course also possible to connect the left-hand end fixedly to the basic body. Pull threads 9 then serve to pull the sheath over balloon 3 and the threads 8 to retract it.

In the catheter 15 in fig. 5 a balloon member 17 is arranged round the distal end of the basic body 16. In a manner corresponding with the foregoing embodiment a protection sheath 18 is arranged which is connected with its relatively distal end 19 to basic body 16. Connected to the relatively proximal end of the protection sheath 18 are pull threads 24 which are guided through orifices 23 in the basic body 16. Pull threads 24 extend through the lumen of basic body 16 to the proximal end where they can be operated.

Received in the lumen of basic body 16 is an inner tubular member 20, via the lumen 21 of which fluid under pressure can be supplied which can flow via apertures 22 into the interior of balloon member 17 in order to expand it. It will be apparent that the lumen of basic body 16 is closed at the distal end in order to enclose the fluid under pressure.

In the embodiment of the catheter 25 in fig. 6 and 7 the protection sheath 26 is arranged on the relatively proximal side of the balloon member. The end 27 of protection sheath 26 remote from the balloon member, that is, the relatively proximal end thereof, is again fixedly connected to the basic body and the sheath 26 can be pulled over the balloon using pull threads 28 which are fastened to the relatively distal end of protection sheath 26 and which run first in distal direction and at the end of catheter 25 are diverted into the lumen of catheter 25 in order to extend through this lumen from the distal end to the proximal end. The attendant physician can operate the pull threads 28 at the proximal end to pull the protection sheath 26 over the balloon.

The pull threads 28 are more particularly received in the lumen of an inner tubular member 29 which is sealingly connected with its distal end part to the wall of the lumen of the basic body. A fluid under pressure for expanding the balloon member can be supplied via the annular channel 30 remaining between the wall of the lumen of the basic body and the inner tubular member.

Fig. 7 shows the catheter of fig. 6 with the balloon in expanded state. Via the annular channel 30 fluid under pressure is fed into the interior of the balloon in order to expand it.

According to another embodiment (not shown), the balloon protection sheath can be accommodated in the position of non-use in the catheter basic body. This can be in a lumen or a cavity specially formed for this purpose. When the sheath is placed in the position of use it is then pulled outward and over the balloon with the pull thread or threads.

## Claims

1. Catheter comprising a tubular basic body (2, 16) with a distal and a proximal end, wherein a balloon member (3) is arranged on the distal end, and close to the balloon member (3) is arranged an elastic balloon protection sheath (6) characterized in that the elastic balloon protection sheath (6) can be pulled over the balloon (3) with at least one pull thread (8, 9) connected to the end of the sheath (6) facing toward the balloon and extending to the proximal end of the catheter.

2. Catheter as claimed in claim 1, wherein the balloon protection sheath (6) is elastically stretchable in longitudinal direction and is fixedly connected with the end remote from the balloon (3) to the basic body (2).

3. Catheter as claimed in claim 1 or 2, wherein the basic body comprises at least one lumen (21) and the pull thread (8, 9) is guided through the lumen.

4. Catheter as claimed in claim 3, wherein the balloon protection sheath (6) is arranged close to the relatively proximal end of the balloon member (3) and the pull thread (8) first extends from the balloon protection sheath (6) in distal direction, is guided to the lumen through an orifice at the distal end of the basic body and subsequently extends through the lumen to the proximal end.

5. Catheter as claimed in any of the foregoing claims, comprising pull threads (8, 9) acting in opposing directions.

6. Catheter as claimed in any of the foregoing claims, wherein the balloon protection sheath (6) is of woven material.

## Patentansprüche

1. Katheter mit einem schlauchförmigen Grundkörper (2, 16) mit einem distalen und einem proximalen Ende, wobei ein Ballonkörper (3) am distalen Ende angeordnet ist und benachbart zum Ballonkörper (3) eine elastische Ballon-Schutzumhüllung (6) befindlich ist,
dadurch gekennzeichnet, daß die elastische Ballon-Schutzumhüllung (6) über den Ballon (3) mittels wenigstens eines Zugfadens (8, 9) gezogen werden kann, der an jenem Ende der Umhüllung (6) befestigt ist, das dem Ballon zugewandt ist und sich nach dem proximalen Ende des Katheters erstreckt.

2. Katheter nach Anspruch 1, bei welchem die Ballon-Schutzumhüllung (6) elastisch in Längsrichtung streckbar und fest mit dem Ende des Grundkörpers (2) verbunden ist, welches dem Ballon (3) abgewandt ist.

3. Katheter nach den Ansprüchen 1 oder 2, bei welchem der Grundkörper wenigstens einen Hohlraum (21) aufweist und der Zugfaden (8, 9) durch diesen Hohlraum geführt ist.

4. Katheter nach Anspruch 3, bei welchem die Ballon-Schutzumhüllung (6) benachbart zu dem relativ proximalen Ende des Ballonkörpers (3) angeordnet ist und der Zugfaden (8) sich zunächst von der Ballon-Schutzumhüllung (6) in distaler Richtung erstreckt und nach dem Hohlraum über eine Öffnung am distalen Ende des Grundkörpers geführt ist und sich danach durch den Hohlraum nach dem proximalen Ende erstreckt.

5. Katheter nach einem der vorhergehenden Ansprüche, welcher Zugfäden (8, 9) aufweist, die in entgegengesetzter Richtung wirken.

6. Katheter nach einem der vorhergehenden Ansprüche, bei welchem die Ballon-Schutzumhüllung (6) aus gewebtem Material besteht.

## Revendications

1. Cathéter comprenant un corps de base tubulaire (2, 16) avec une extrémité distale et une extrémité proximale, dans lequel un organe formant ballonnet (3) est agencé sur l'extrémité distale, et, à proximité de l'organe ballonnet (3), est agencée une gaine de protection (6) élastique de ballonnet, caractérisé en ce que la gaine de protection (6) élastique de ballonnet peut être tirée au-dessus du ballonnet (3) avec au moins un fil de traction (8, 9) relié à l'extrémité de la gaine (6) faisant face au ballonnet et s'étendant vers l'extrémité proximale du cathéter.

2. Cathéter selon la revendication 1, dans lequel la gaine de protection de ballonnet (6) peut être étirée de manière élastique dans la direction longitudinale et est reliée à demeure par l'extrémité distante par rapport au ballonnet (3) au corps de base (2).

3. Cathéter selon la revendication 1 ou 2, dans lequel le corps de base comprend au moins une lumière (21) et le fil de traction (8, 9) est guidé à travers la lumière.

4. Cathéter selon la revendication 3, dans lequel la gaine de protection de ballonnet (6) est agencée à proximité de l'extrémité relativement proximale de l'organe ballonnet (3) et le fil de traction (9) s'étend d'abord à partir de la gaine de protection de ballonnet (6) en direction distale, est guidé vers la lumière à travers un orifice à l'extrémité distale du corps de base et s'étend ensuite à travers la lumière vers l'extrémité proximale.

5. Cathéter selon l'une quelconque des revendications précédentes, comprenant des fils de traction (8, 9) agissant dans des sens opposés.

6. Cathéter selon l'une quelconque des revendications précédentes, dans lequel la gaine de protection de ballonnet (6) est en un matériau tissé.
